(19) Europäisches Patentamt
European Patent Office
Office européen des brevets

(11) EP 4 212 625 A1

(12) EUROPEAN PATENT APPLICATION
published in accordance with Art. 153(4) EPC

(43) Date of publication:
**19.07.2023 Bulletin 2023/29**

(21) Application number: **21866528.9**

(22) Date of filing: **24.08.2021**

(51) International Patent Classification (IPC):
***C12P 13/00*** (2006.01) ***C12N 1/21*** (2006.01)
***C12N 15/09*** (2006.01) ***C12N 15/53*** (2006.01)
***C12N 15/63*** (2006.01) ***C12N 9/02*** (2006.01)

(52) Cooperative Patent Classification (CPC):
**C12N 9/0004; C12N 15/09; C12N 15/63; C12N 15/74; C12P 13/00**

(86) International application number:
**PCT/JP2021/031005**

(87) International publication number:
**WO 2022/054568 (17.03.2022 Gazette 2022/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.09.2020 JP 2020153992**

(71) Applicant: **Kao Corporation**
**Chuo-ku**
**Tokyo 103-8210 (JP)**

(72) Inventors:
- **NONAKA, Kyoshiro**
**Wakayama-shi, Wakayama 640-8580 (JP)**
- **TAKAHASHI, Fumikazu**
**Wakayama-shi, Wakayama 640-8580 (JP)**

(74) Representative: **Vossius & Partner**
**Patentanwälte Rechtsanwälte mbB**
**Siebertstraße 3**
**81675 München (DE)**

Remarks:
The complete document including Reference Table(s) and the Sequence Listing(s) can be downloaded from the EPO website

# (54) POLYPEPTIDE HAVING 4-AMINOBENZOIC ACID HYDROXYLATION ACTIVITY AND USE THEREOF

(57) Provided are a polypeptide having excellent 4-aminobenzoic acid hydroxylation activity and a method for using the same. The present invention provides a polypeptide having 4-aminobenzoic acid hydroxylation activity, consisting of the amino acid sequence represented by SEQ ID NO: 2 or an amino acid sequence having at least 90% identity thereto, and having an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 being the following amino acid: (a) the position 39 or the position corresponding thereto: cysteine, (b) the position 43 or the position corresponding thereto: leucine or histidine, (c) the position 83 or the position corresponding thereto: valine, glycine, or phenylalanine, (d) the position 106 or the position corresponding thereto: alanine, cysteine, isoleucine, or threonine, (e) the position 180 or the position corresponding thereto: alanine, (f) the position 266 or the position corresponding thereto: valine or phenylalanine, (g) the position 346 or the position corresponding thereto: leucine, (h) the position 363 or the position corresponding thereto: valine or leucine, and (i) the position 371 or the position corresponding thereto: phenylalanine.

EP 4 212 625 A1

## Description

Field of the Invention

The present invention relates to a polypeptide having 4-aminobenzoic acid hydroxylation activity and use thereof.

Background of the Invention

Polybenzoxazole (PBO) is known as an engineering plastic excellent in heat resistance and mechanical strength, and used in fiber materials and insulating films of semiconductor devices and the like. (Non Patent Literature 1).

A benzoxazole backbone was formed by the condensation of an o-aminophenol backbone with carboxylic acid. Hence, it is expected that 4-amino-3-hydroxybenzoic acids (4,3-AHBAs) having these functional groups in their molecules are useful as PBO monomers. In actuality, the synthesis and physical property evaluation of polybenzoxazole using 4,3-AHBA have been studied (Non Patent Literature 2).

Methods for producing compounds by microbial fermentation using renewable sources as starting materials have received attention in recent years for reduction in global environmental load and the like. For example, the microbial production and polymerization of 3-amino-4-hydroxybenzoic acid (3,4-AHBA), which is structurally similar to 4,3-AHBA, have been studied (Patent Literature 1).

As for the production of 4,3-AHBA, for example, a synthesis method of chemically reducing a nitro aromatic compound has been known so far (Patent Literature 2). A possible strategy which enables fermentative production of 4,3-AHBA by a microbial method is the hydroxylation at position 3 of 4-aminobenzoic acid (4-ABA) which can be biosynthesized within a microbe. However, for such reaction, it has merely been reported that some 4-hydroxybenzoic acid hydroxylases have slight activity (Non Patent Literatures 3 and 4).

[Patent Literature 1] JP-B-5445453
[Patent Literature 2] JP-B-3821350
[Non Patent Literature 1] Hiroki Murase, SENI GAKKAISHI (Seni To Kogyo, Journal of Fiber Science and Technology), Vol. 66, No. 6 (2010)
[Non Patent Literature 2] Lon J. Mathias et al., Macromolecules, Vol. 18, No. 4, pp. 616-622 (1985)
[Non Patent Literature 3] Barrie Entsch et al., The Journal of Biological Chemistry, Vol. 262, No. 13, pp. 6060-6068 (1987)
[Non Patent Literature 4] Domenico L. Gatti et al., Biochemistry, Vol. 35, No. 2, pp. 567-578 (1996)

Summary of the Invention

The present invention relates to the following 1) to 7) .

1) A polypeptide having 4-aminobenzoic acid hydroxylation activity, consisting of the amino acid sequence represented by SEQ ID NO: 2 or an amino acid sequence having at least 90% identity thereto, and having an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 being the following amino acid:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

2) A method for producing a mutant polypeptide having 4-aminobenzoic acid hydroxylation activity, the polypeptide consisting of the amino acid sequence represented by SEQ ID NO: 2 or an amino acid sequence having at least 90% identity thereto, having 4-aminobenzoic acid hydroxylation activity, and having an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 being the following amino acid, in a

polypeptide consisting of the amino acid sequence represented by SEQ ID NO: 2 or an amino acid sequence having at least 90% identity thereto:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

3) A method for improving 4-aminobenzoic acid hydroxylation activity, comprising substituting an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 with the following amino acid, in a polypeptide consisting of the amino acid sequence represented by SEQ ID NO: 2 or an amino acid sequence having at least 90% identity thereto, and having 4-aminobenzoic acid hydroxylation activity:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

4) A polynucleotide encoding the polypeptide according to 1).
5) A vector or a DNA fragment comprising the polynucleotide according to 4).
6) A transformed cell comprising the vector or the DNA fragment according to 5).
7) A method for producing 4-amino-3-hydroxybenzoic acids, comprising a step of culturing the transformed cell according to 6).

Detailed Description of the Invention

The present invention relates to a provision of a polypeptide having excellent 4-aminobenzoic acid hydroxylation activity and a method for using the same.
The present inventors found that a 4-hydroxybenzoic acid hydroxylase mutant having a particular amino acid sequence has excellent 4-aminobenzoic acid hydroxylation activity and is useful in the production of 4-amino-3-hydroxybenzoic acids.
Since the polypeptide having 4-aminobenzoic acid hydroxylation activity according to the present invention has excellent 4-aminobenzoic acid hydroxylation activity, use thereof enables 4-amino-3-hydroxybenzoic acids to be efficiently produced from 4-aminobenzoic acids.
In the present specification, the identity of an amino acid sequence or a nucleotide sequence is calculated by the Lipman-Pearson method (Science, 1985, 227: 1435-1441). Specifically, the identity is calculated by analysis with a unit size to compare (ktup) set to 2 using the homology analysis (search homology) program of genetic information processing software GENETYX Ver. 12.
In the present specification, the "position corresponding" on an amino acid sequence or a nucleotide sequence can be determined by aligning a sequence of interest and a reference sequence (e.g., the amino acid sequence represented by SEQ ID NO: 2) so as to provide the maximum homology. The alignment of amino acid sequences or nucleotide sequences can be carried out using an algorithm known in the art, and the procedures thereof are known to those skilled in the art. The alignment can be performed, for example, by using default settings of Clustal W multiple alignment program (Thompson, J.D. et al., 1994, Nucleic Acids Res. 22: 4673-4680). Alternatively, Clustal W2 or Clustal omega, a modified version of Clustal W, may be used. Clustal W, Clustal W2 and Clustal omega are available on, for example, the website of European Bioinformatics Institute (EBI [www.ebi.ac.uk/index.html]) or the DNA Databank of Japan (DDBJ [www.ddbj.nig.ac.jp/searches-j.html]) run by National Institute of Genetics. A position of the sequence of interest aligned to an

arbitrary position of the reference sequence by the alignment mentioned above is regarded as a "position corresponding" to the arbitrary position.

Those skilled in the art can further finely adjust the alignment of amino acid sequences obtained as described above for optimization. Such optimum alignment is preferably determined in consideration of the similarity between the amino acid sequences, the frequency of a gap to be inserted and the like. In this context, the similarity between the amino acid sequences refers the ratio (%) of the number of positions at which identical or similar amino acid residues are present in both the sequences to the number of full-length amino acid residues when these two amino acid sequences are aligned. The similar amino acid residues mean amino acid residues which are similar in property to each other in terms of polarity or electric charge and cause so-called conservative substitution, among 20 amino acids constituting a protein. Groups consisting of such similar amino acid residues are well known to those skilled in the art. Examples thereof include, but are not limited to: arginine and lysine or glutamine; glutamic acid and aspartic acid or glutamine; serine and threonine or alanine; glutamine and asparagine or arginine; and leucine and isoleucine.

In the present specification, the "amino acid residue" means any of 20 amino acid residues constituting a protein, i.e., alanine (Ala or A), arginine (Arg or R), asparagine (Asn or N), aspartic acid (Asp or D), cysteine (Cys or C), glutamine (Gln or Q), glutamic acid (Glu or E), glycine (Gly or G), histidine (His or H), isoleucine (Ile or I), leucine (Leu or L), lysine (Lys or K), methionine (Met or M), phenylalanine (Phe or F), proline (Pro or P), serine (Ser or S), threonine (Thr or T), tryptophan (Trp or W), tyrosine (Tyr or Y) and valine (Val or V).

In the present specification, the "operable linkage" of a gene to a control region such as a promoter means that the gene is linked to the control region such that the gene is expressible under the control of the control region. The procedures of the "operable linkage" of a gene to a control region are well known to those skilled in the art.

In the present specification, the terms "upstream" and "downstream" in relation to a gene refer to upstream and downstream in the direction of transcription of the gene. For example, the phrase "gene located downstream of a promoter" means that the gene resides on the 3' side of the promoter in a DNA sense strand, and the phrase "upstream of a gene" means a region on the 5' side of the gene in a DNA sense strand.

In the present specification, the term "original" which is used for a function, property, or trait of a cell is used for indicating that the function, the property, or the trait is indigenous to the cell. By contrast, the term "foreign" is used for indicating that the function, the property, or the trait is introduced ab *extra,* not indigenous to the cell. For example, a "foreign" gene or polynucleotide is a gene or a polynucleotide introduced *ab extra* to a cell. The foreign gene or polynucleotide may be derived from an organism of the same species as that of the cell in to which the gene or polynucleotide is introduced or may be derived from an organism of different species therefrom (i.e., a heterologous gene or polynucleotide).

<Polypeptide having 4-aminobenzoic acid hydroxylation activity>

The polypeptide having 4-aminobenzoic acid hydroxylation activity according to the present invention (referred to as the "polypeptide of the present invention") is a polypeptide consisting of the amino acid sequence represented by SEQ ID NO: 2 or an amino acid sequence having at least 90% identity thereto, and having an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 being the following amino acid:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

The polypeptide is a mutant polypeptide having 4-aminobenzoic acid hydroxylation activity, which consists of a polypeptide serving as a reference, i.e., a polypeptide consisting of the amino acid sequence represented by SEQ ID NO: 2 or an amino acid sequence having at least 90% identity thereto, and in which an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 is substituted with any of the amino acids (a) to (i).

In the present invention, the "4-aminobenzoic acid hydroxylation activity" means activity of catalyzing the hydroxylation of 4-aminobenzoic acids, preferably activity of catalyzing the hydroxylation at position 3 of 4-aminobenzoic acids.

The 4-aminobenzoic acid hydroxylation activity can be determined, as shown in Examples mentioned later, by culturing a microorganism producing the polypeptide of the present invention, and measuring the amount of 4-amino-3-hydroxybenzoic acid produced by HPLC or the like.

The polypeptide of the present invention can be produced by substituting an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 with the following amino acid, in a polypeptide consisting of the amino acid sequence represented by SEQ ID NO: 2 or an amino acid sequence having at least 90% identity thereto, and having 4-aminobenzoic acid hydroxylation activity:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

In this context, the polypeptide consisting of the amino acid sequence represented by SEQ ID NO: 2 or an amino acid sequence having at least 90% identity thereto, and having 4-aminobenzoic acid hydroxylation activity is a "parent" polypeptide of the polypeptide of the present invention.

The "parent" polypeptide refers to a reference polypeptide which becomes the polypeptide of the present invention by introducing a predetermined mutation to the amino acid residue.

In the present invention, HFM122, a polypeptide consisting of the amino acid sequence represented by SEQ ID NO: 2 (NCBI Reference Sequence: WP_010920262.1), is known as 4-hydroxybenzoate 3-monooxygenase (EC1.14.13.2). The 4-hydroxybenzoate 3-monooxygenase is an enzyme having catalytic activity of accelerating any one or both of reaction to produce protocatechuic acid by hydroxylating position 3 of 4-hydroxybenzoic acid and inverse reaction thereof, and is an enzyme which catalyzes the hydroxylation of 4-hydroxybenzoic acids (4-hydroxybenzoic acid hydroxylase).

This HFM122 was found to have 4-aminobenzoic acid hydroxylation activity by the present applicant (Japanese Patent Application No. 2018-171849).

Examples of the polypeptide consisting of an amino acid sequence having at least 90% identity to the amino acid sequence represented by SEQ ID NO: 2, and having 4-aminobenzoic acid hydroxylation activity include a polypeptide which consists of an amino acid sequence having at least 90% identity, specifically, 90% or higher, preferably 95% or higher, more preferably 96% or higher, further preferably 97% or higher, further preferably 98% or higher, further preferably 99% or higher identity to the amino acid sequence represented by SEQ ID NO: 2, and has 4-aminobenzoic acid hydroxylation activity.

The parent polypeptide preferably has a valine residue at position 39 of the amino acid sequence represented by SEQ ID NO: 2 or a position corresponding thereto, preferably has a valine residue at position 43 thereof or a position corresponding thereto, preferably has a methionine residue at position 83 thereof or a position corresponding thereto, preferably has a methionine residue at position 106 thereof or a position corresponding thereto, preferably has a valine residue at position 180 thereof or a position corresponding thereto, preferably has an alanine residue at position 266 thereof or a position corresponding thereto, preferably has a phenylalanine residue at position 346 thereof or a position corresponding thereto, preferably has a methionine residue at position 363 thereof or a position corresponding thereto, or preferably has an isoleucine residue at position 371 thereof or a position corresponding thereto. For the polypeptide of the present invention, it is more preferred to substitute valine at the position 39 or the position corresponding thereto with cysteine, to substitute valine at the position 43 or the position corresponding thereto with leucine or histidine, to substitute methionine at the position 83 or the position corresponding thereto with valine, glycine or phenylalanine, to substitute methionine at the position 106 or the position corresponding thereto with alanine, cysteine, isoleucine or threonine, to substitute valine at the position 180 or the position corresponding thereto with alanine, to substitute alanine at the position 266 or the position corresponding thereto with valine or phenylalanine, to substitute phenylalanine at the position 346 or the position corresponding thereto with leucine, to substitute methionine at the position 363 or the position corresponding thereto with valine or leucine, or to substitute isoleucine at the position 371 or the position corresponding thereto with phenylalanine. It is further preferred to substitute valine at the position 43 or the position corresponding thereto with histidine, to substitute methionine at the position 106 or the position corresponding thereto with alanine, isoleucine or threonine, to substitute valine at the position 180 or the position corresponding thereto with alanine, to substitute alanine at the position 266 or the position corresponding thereto with valine, to substitute phenylalanine at

the position 346 or the position corresponding thereto with leucine, to substitute methionine at the position 363 or the position corresponding thereto with leucine, or to substitute isoleucine at the position 371 or the position corresponding thereto with phenylalanine.

<Polynucleotide encoding polypeptide of present invention>

In the present invention, any of various mutagenesis techniques known in the art can be used as an approach of mutating the amino acid residue of the parent polypeptide. For example, a nucleotide sequence encoding the amino acid residue to be mutated in a polynucleotide encoding the amino acid sequence of the parent polypeptide (hereinafter, also referred to as a parent gene) can be mutated to a nucleotide sequence encoding a mutated amino acid residue to obtain a polynucleotide encoding the polypeptide of the present invention.

The introduction of the mutation of interest to the parent gene can be basically performed by use of any of various site-directed mutagenesis methods well known to those skilled in the art. The site-directed mutagenesis method can be performed by, for example, any approach such as inverse PCR or annealing. A commercially available kit for site-directed mutagenesis (e.g., QuikChange II Site-Directed Mutagenesis Kit or QuikChange Multi Site-Directed Mutagenesis Kit from Agilent Technologies, Inc.) may be used.

The site-directed mutagenesis of the parent gene can be performed most generally using primers for mutations containing a nucleotide mutation to be introduced. The primers for mutations can be designed so as to contain a nucleotide sequence which is annealed to a region containing a nucleotide sequence encoding the amino acid residue to be mutated in the parent gene, and has a nucleotide sequence (codon) encoding the mutated amino acid residue instead of the nucleotide sequence (codon) encoding the amino acid residue to be mutated. The nucleotide sequences (codons) encoding the unmutated or mutated amino acid residues can be appropriately recognized and selected by those skilled in the art on the basis of a usual textbook or the like. Alternatively, the site-directed mutagenesis may employ a method of linking DNA fragments respectively amplified from the upstream and downstream sides of a mutation site by separately using two complementary primers containing a nucleotide mutation to be introduced, into one fragment by SOE (splicing by overlap extension)-PCR (Gene, 1989, 77 (1): p. 61-68).

Template DNA containing the parent gene can be prepared by extracting genomic DNA by a routine method from a microorganism producing the 4-hydroxybenzoic acid hydroxylase mentioned above, or by extracting RNA therefrom and synthesizing cDNA by reverse transcription. Alternatively, a corresponding nucleotide sequence may be chemically synthesized on the basis of the amino acid sequence of the parent polypeptide, and used as template DNA. DNA sequences containing nucleotide sequences encoding HFM122 already mentioned as polypeptides having 4-aminobenzoic acid hydroxylation activity are shown in SEQ ID NO: 1.

The primers for mutations can be prepared by a well-known oligonucleotide synthesis method such as phosphoramidite method (Nucleic Acids R4esearch, 1989, 17: 7059-7071). Such primer synthesis may be carried out using, for example, a commercially available oligonucleotide synthesis apparatus (manufactured by Applied Biosystems Inc. (ABI) or the like.). The polynucleotide encoding the polypeptide of the present invention having the mutation of interest can be obtained by the site-directed mutagenesis as described above with the parent gene as template DNA using a primer set containing the primers for mutations.

The polynucleotide encoding the polypeptide of the present invention may contain single-stranded or doublestranded DNA, cDNA, RNA or other artificial nucleic acids. The DNA, the cDNA and the RNA may be chemically synthesized. The polynucleotide may also contain an open reading frame (ORF) as well as the nucleotide sequence of an untranslated region (UTR). The polynucleotide may be codon-optimized for the species of the transformant for mutant polypeptide production of the present invention. Information on codons which are used by various organisms is available from Codon Usage Database
([www.kazusa.or.jp/codon/]).

<Vector or DNA fragment>

The obtained polynucleotide encoding the polypeptide of the present invention can be inserted to a vector.

The vector containing the polynucleotide is an expression vector. Preferably, the vector is an expression vector which can introduce the polynucleotide encoding the polypeptide of the present invention into a host microorganism and enables the polynucleotide to be expressed within the host microorganism. Preferably, the vector contains the polynucleotide encoding the polypeptide of the present invention, and a control region operably linked thereto. The vector may be a vector capable of proliferating and replicating autonomously outside the chromosome (i.e., in a plasmid), or may be a vector which is integrated into the chromosome.

Specific examples of the vector include pBluescript II SK(-) (Stratagene California), pUC series vectors such as pUC18/19 and pUC118/119 (Takara Bio Inc.), pET series vectors (Takara Bio Inc.), pGEX series vectors (GE Healthcare Japan Corp.), pCold series vectors (Takara Bio Inc.), pHY300PLK (Takara Bio Inc.), pUB110 (Mckenzie, T. et al.,

1986, Plasmid 15 (2): 93-103), pBR322 (Takara Bio Inc.), pRS403 (Stratagene California), pMW218/219 (Nippon Gene Co., Ltd.), pRI series vectors such as pRI909/910 (Takara Bio Inc.), pBI series vectors (Clontech Laboratories, Inc.), IN3 series vectors (Inplanta Innovations Inc.), pPTR1/2 (Takara Bio Inc.), pDJB2 (D.J. Ballance et al., Gene, 36, 321-331, 1985), pAB4-1 (van Hartingsveldt W et al., Mol Gen Genet, 206, 71-75, 1987), pLeu4 (M.I.G. Roncero et al., Gene, 84, 335-343, 1989), pPyr225 (C.D. Skory et al., Mol Genet Genomics, 268, 397-406, 2002), and pFG1 (Gruber, F. et al., Curr Genet, 18, 447-451, 1990).

The polynucleotide encoding the polypeptide of the present invention may be constructed as a DNA fragment containing the same. Examples of the DNA fragment include PCR-amplified DNA fragments and restriction enzyme-cleaved DNA fragments. Preferably, the DNA fragment can be an expression cassette containing the polynucleotide encoding the polypeptide of the present invention, and a control region operably linked thereto.

The control region contained in the vector or the DNA fragment is a sequence for allowing the polynucleotide encoding the polypeptide of the present invention to be expressed within a host cell into which the vector or the DNA fragment is introduced. Examples thereof include expression regulation regions such as promoters and terminators, and replication origins. The type of the control region can be appropriately selected according to the type of the host microorganism into which the vector or the DNA fragment is introduced. If necessary, the vector or the DNA fragment may further have a selective marker such as an antibiotic resistance gene or an amino acid synthesis-related gene (e.g., a resistance gene for a drug such as ampicillin, neomycin, kanamycin, or chloramphenicol).

The vector or the DNA fragment may contain a polynucleotide sequence encoding a polypeptide necessary for biosynthesizing 4-aminobenzoic acids. Examples of the polypeptide necessary for biosynthesizing 4-aminobenzoic acids include 4-amino-4-deoxychorismate synthase (pabAB) and 4-amino-4-deoxychorismate lyase (pabC).

The linkage of the polynucleotide encoding the polypeptide of the present invention to the control region or the marker gene sequence can be performed by a method such as the SOE-PCR mentioned above. Procedures of introducing the gene sequence to the vector are well known in the art. The type of the control region such as a promoter region, a terminator, or a secretion signal region is not particularly limited, and a promoter or a secretion signal sequence usually used can be appropriately selected and used according to the recipient host.

Preferred examples of the control region include, but are not particularly limited to, strong control regions which can enhance expression as compared with a wild type, for example, high-expression promoters known in the art such as T7 promoter, lac promoter, tac promoter, and trp promoter.

transformed cell>

The transformed cell of the present invention can be obtained by introducing the vector containing the polynucleotide encoding the polypeptide of the present invention into a host, or by introducing the DNA fragment containing the polynucleotide encoding the polypeptide of the present invention into the genome of a host.

The transformed cell is a cell into which the polynucleotide encoding the polypeptide of the present invention is introduced to express it, and can be a cell having the enhanced expression of the polynucleotide, and by extension, a cell having the enhanced expression of the polypeptide of the present invention.

Any of cells of a fungi, a yeast, actinomycete, *E. coli, Bacillus subtilis,* or the like may be used as a host cell, and *E. coli* or actinomycete is preferred. Examples of the actinomycete include a bacterium of the genus *Corynebacterium,* a bacterium of the genus *Mycobacterium,* a bacterium of the genus *Rhodococcus,* a bacterium of the genus *Streptomyces,* and a bacterium of the genus *Propionibacterium.* A bacterium of the genus *Corynebacterium* is preferred, and *Corynebacterium glutamicum* is more preferred.

Among others, a microorganism which can supply 4-aminobenzoic acids serving as substrates in the biosynthesis of 4-amino-3-hydroxybenzoic acids is preferred, and a microorganism having enhanced ability to supply 4-aminobenzoic acids is more preferred. Examples of the method for enhancing the ability of the microorganism to supply 4-aminobenzoic acids include a method of introducing, into the microorganism, a vector containing a polynucleotide encoding a polypeptide necessary for biosynthesizing 4-aminobenzoic acids, and a control region operably linked thereto, and a method of substituting the control region of a polynucleotide encoding a polypeptide necessary for biosynthesizing 4-aminobenzoic acids, which is originally carried by the microorganism, with a strong-expression promoter.

Examples of the method for introducing the vector or the DNA fragment into the host include, for example, electroporation, transformation, transfection, conjugation, protoplast method, particle gun method, or Agrobacterium method.

Examples of the method for introducing the polynucleotide into the host include, but are not particularly limited to, a double crossover method using the DNA fragment containing the polynucleotide. The DNA fragment may be introduced into the downstream of a promoter sequence of a gene having a large expression level in the host cell mentioned above, or a fragment of the DNA fragment operably linked to the control region mentioned above may be prepared in advance, and the linked fragment can be introduced into the genome of the host. The DNA fragment may be further linked in advance to a marker (drug resistance gene, auxotrophic complementary gene and the like) for

selecting a cell into which the polynucleotide of the present invention is introduced properly.

The transformed cell into which the vector or the DNA fragment of interest is introduced can be selected by exploiting the selective marker. When the selective marker is, for example, an antibiotic resistance gene, the transformed cell into which the vector or the DNA fragment of interest is been introduced can be selected by culture in a culture medium supplemented with the antibiotic. When the selective marker is, for example, an amino acid synthesis-related gene, the transformed cell into which the vector or the DNA fragment of interest is introduced can be selected by using the presence or absence of the amino acid auxotrophy as an index after introduction of gene into a host microorganism requiring the amino acid. Alternatively, the introduction of the vector or the DNA fragment of interest may be confirmed by examining the DNA sequence of the transformed cell by PCR or the like.

The transformed cell obtained above is cultured in a proper culture medium so that the polynucleotide introduced into the cell is expressed to produce the polypeptide of the present invention. Specifically, the transformed cell is capable of serving as a bacterium producing a polypeptide having 4-aminobenzoic acid hydroxylation activity. In the case of culturing the transformed cell of the present invention, as shown in Examples mentioned later, the productivity of 4-amino-3-hydroxybenzoic acid is improved as compared with the case of using a transformed cell producing the parent polypeptide.

Specifically, in a polypeptide consisting of the amino acid sequence represented by SEQ ID NO: 2 or an amino acid sequence having at least 90% identity thereto, and having 4-aminobenzoic acid hydroxylation activity, the mutation to substitute an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 with the following amino acid is useful in improvement in 4-aminobenzoic acid hydroxylation activity and also useful in improvement in the productivity of 4-amino-3-hydroxybenzoic acids:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

Furthermore, the transformed cell of the present invention serves as a bacterium producing a polypeptide having improved 4-aminobenzoic acid hydroxylation activity and serves as a useful strain producing 4-amino-3-hydroxybenzoic acids.

<Production of 4-amino-3-hydroxybenzoic acids>

The method for producing 4-amino-3-hydroxybenzoic acids of the present invention includes a step of culturing the transformed cell of the present invention, and 4-amino-3-hydroxybenzoic acids can be obtained by collecting the 4-amino-3-hydroxybenzoic acids from a culture medium.

In the present invention, specific examples of the 4-amino-3-hydroxybenzoic acids include 4-amino-3-hydroxybenzoic acid derivatives of the following formula (1) :

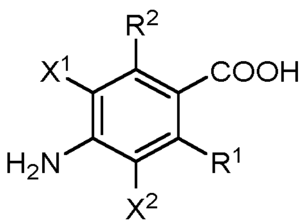

(1)

wherein $R^1$ represents a hydrogen atom, a hydroxy group (-OH), a methoxy group ($-OCH_3$), an amino group ($-NH_2$), a fluorine atom (-F), a chlorine atom (-Cl), a bromine atom (-Br), an iodine atom (-I), a carboxy group (-COOH), a methyl group ($-CH_3$), or an ethyl group ($-CH_2CH_3$), $R^2$ represents a hydrogen atom, a hydroxy group (-OH), a methoxy group ($-OCH_3$), an amino group ($-NH_2$), a fluorine atom (-F), a chlorine atom (-Cl), a bromine atom (-Br), an iodine atom (-I), a carboxy group (-COOH), a methyl group ($-CH_3$) or an ethyl group ($-CH_2CH_3$), and $X^1$ and $X^2$ each represent a hydrogen

atom or a hydroxy group, at least one of which represents a hydroxy group.

The functional group $R^1$ is preferably a hydrogen atom, a hydroxy group (-OH), a methoxy group ($-OCH_3$), a fluorine atom (-F) or a methyl group ($-CH_3$).

The functional group $R^2$ is preferably a hydrogen atom, a hydroxy group (-OH), a methoxy group ($-OCH_3$), a fluorine atom (-F) or a methyl group ($-CH_3$).

More preferably, both $R^1$ and $R^2$ are hydrogen atoms.

Both $X^1$ and $X^2$ may be hydroxy groups, and either one of $X^1$ or $X^2$ is preferably a hydroxy group.

The culture medium can contain, if necessary, 4-aminobenzoic acids serving as substrates in the biosynthesis of 4-amino-3-hydroxybenzoic acids.

In this context, examples of the 4-aminobenzoic acids include 4-aminobenzoic acid derivatives of the following formula (2):

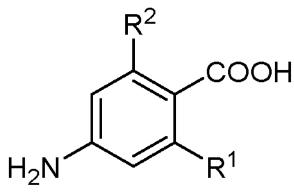

(2)

wherein $R^1$ and $R^2$ are as defined above.

Any of a natural culture medium and a synthetic culture medium may be used as the culture medium for the culture of the transformed cell as long as the culture medium contains a carbon source, a nitrogen source, inorganic salts and the like and permits efficient culture of the transformed cell of the present invention. For example, saccharides such as glucose, polyols such as glycerin, alcohols such as ethanol, or organic acids such as pyruvic acid, succinic acid or citric acid can be used as carbon sources. For example, peptone, meat extracts, yeast extracts, casein hydrolysates, alkali extracts of soybean meal, alkylamines such as methylamine, or ammonia or its salt can be used as nitrogen sources. In addition, phosphate, carbonate, sulfate, salts of magnesium, calcium, potassium, iron, manganese, zinc, or the like, a particular amino acid, a particular vitamin, an antifoaming agent or the like may be used, if necessary.

The culture can usually be performed, if necessary, with stirring or shaking, at 10°C to 40°C for 6 hours to 72 hours, preferably for 9 hours to 60 hours, more preferably for 12 hours to 48 hours. During the culture, an antibiotic such as ampicillin or kanamycin may be added, if necessary, to the culture medium.

The methods for collecting and purifying 4-amino-3-hydroxybenzoic acids from the cultures are not particularly limited. Specifically, the collection and the purification can be carried out by combining a well-known ion-exchange resin method, precipitation method, crystallization method, recrystallization method, concentration method and other methods. For example, after removal of bacterial cells by centrifugation or the like, ionic substances are removed with cation- and anion-exchange resins, and the resultant can be concentrated to obtain 4-amino-3-hydroxybenzoic acids. The 4-amino-3-hydroxybenzoic acids accumulated in the cultures may be used directly without being isolated.

The present invention also encompasses the following substances, production methods, use, methods and the like as exemplary embodiments. However, the present invention is not limited by these embodiments.

<1> A polypeptide having 4-aminobenzoic acid hydroxylation activity, consisting of the amino acid sequence represented by SEQ ID NO: 2 or an amino acid sequence having at least 90% identity thereto, and having an amino acid residue at position 39, 43, 83, 106, 180, 266, 346, 363 or 371 of the amino acid sequence represented by SEQ ID NO: 2, or a position corresponding to the position 39, 43, 83, 106, 180, 266, 346, 363 or 371 being the following amino acid:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

<2> A mutant polypeptide having 4-aminobenzoic acid hydroxylation activity, consisting of the amino acid sequence

represented by SEQ ID NO: 2 or an amino acid sequence having at least 90% identity thereto, wherein an amino acid residue at position 39, 43, 83, 106, 180, 266, 346, 363 or 371 of the amino acid sequence represented by SEQ ID NO: 2, or a position corresponding to the position 39, 43, 83, 106, 180, 266, 346, 363 or 371 is substituted with the following amino acid:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

<3> The mutant polypeptide according to <2>, wherein the substitution of amino acid residue is substitution of valine at the position 43 or the position corresponding thereto with histidine, substitution of methionine at the position 106 or the position corresponding thereto with alanine, isoleucine or threonine, substitution of valine at the position 180 or the position corresponding thereto with alanine, substitution of alanine at the position 266 or the position corresponding thereto with valine, substitution of phenylalanine at the position 346 or the position corresponding thereto with leucine, substitution of methionine at the position 363 or the position corresponding thereto with leucine, or substitution of isoleucine at the position 371 or the position corresponding thereto with phenylalanine.
<4> A method for producing a mutant polypeptide having 4-aminobenzoic acid hydroxylation activity, comprising substituting an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 with the following amino acid, in a polypeptide consisting of the amino acid sequence represented by SEQ ID NO: 2 or an amino acid sequence having at least 90% identity thereto, and having 4-aminobenzoic acid hydroxylation activity:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

<5> The method according to <4>, wherein the substitution of amino acid residue is substitution of valine at the position 43 or the position corresponding thereto with histidine, substitution of methionine at the position 106 or the position corresponding thereto with alanine, isoleucine or threonine, substitution of valine at the position 180 or the position corresponding thereto with alanine, substitution of alanine at the position 266 or the position corresponding thereto with valine, the substitution of phenylalanine at the position 346 or the position corresponding thereto with leucine, the substitution of methionine at the position 363 or the position corresponding thereto with leucine, or the substitution of isoleucine at the position 371 or the position corresponding thereto with phenylalanine.
<6> A method for improving 4-aminobenzoic acid hydroxylation activity, comprising substituting an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 with the following amino acid, in a polypeptide consisting of the amino acid sequence represented by SEQ ID NO: 2 or an amino acid sequence having at least 90% identity thereto, and having 4-aminobenzoic acid hydroxylation activity:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,

(g) the position 346 or the position corresponding thereto: leucine,
(h) the position 363 or the position corresponding thereto: valine or leucine, and
(i) the position 371 or the position corresponding thereto: phenylalanine.

<7> The method according to <6>, wherein the substitution of amino acid residue is substitution of valine at the position 43 or the position corresponding thereto with histidine, substitution of methionine at the position 106 or the position corresponding thereto with alanine, isoleucine or threonine, substitution of valine at the position 180 or the position corresponding thereto with alanine, substitution of alanine at the position 266 or the position corresponding thereto with valine, substitution of phenylalanine at the position 346 or the position corresponding thereto with leucine, substitution of methionine at the position 363 or the position corresponding thereto with leucine, or substitution of isoleucine at the position 371 or the position corresponding thereto with phenylalanine.
<8> A method for improving the productivity of 4-amino-3-hydroxybenzoic acids, comprising substituting an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 with the following amino acid, in the case of producing 4-amino-3-hydroxybenzoic acids using a polypeptide consisting of the amino acid sequence represented by SEQ ID NO: 2 or an amino acid sequence having at least 90% identity thereto, and having 4-aminobenzoic acid hydroxylation activity:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

<9> The method according to <8>, wherein the substitution of amino acid residue is substitution of valine at the position 43 or the position corresponding thereto with histidine, substitution of methionine at the position 106 or the position corresponding thereto with alanine, isoleucine or threonine, substitution of valine at the position 180 or the position corresponding thereto with alanine, substitution of alanine at the position 266 or the position corresponding thereto with valine, substitution of phenylalanine at the position 346 or the position corresponding thereto with leucine, substitution of methionine at the position 363 or the position corresponding thereto with leucine, or substitution of isoleucine at the position 371 or the position corresponding thereto with phenylalanine.
<10> A polynucleotide encoding the polypeptide according to any of <1> to <3>.
<11> A vector or a DNA fragment comprising the polynucleotide according to <10>.
<12> A transformed cell comprising the vector or the DNA fragment according to <11>.
<13> The transformed cell according to <12>, wherein the transformed cell is derived from *E. coli* or a bacterium of the genus *Corynebacterium.*
<14> The transformed cell according to <12> or <13>, wherein the transformed cell is a microorganism capable of supplying 4-aminobenzoic acids.
<15> The transformed cell according to <12> or <13>, wherein the transformed cell has improved ability to supply 4-aminobenzoic acids.
<16> A method for producing 4-amino-3-hydroxybenzoic acids, comprising the step of culturing the transformed cell according to any of <12> to <15>.
<17> The method according to <16>, wherein the culture is performed in a culture medium comprising a saccharide as a carbon source.
<18> The method according to <16> or <17>, further comprising a step of collecting 4-amino-3-hydroxybenzoic acids from a culture medium.
<19> The method according to any of <16> to <18>, wherein the culture is performed in the presence of 4-aminobenzoic acids.
<20> The method according to any of <16> to <19>, wherein the 4-amino-3-hydroxybenzoic acids are 4-amino-3-hydroxybenzoic acid derivatives of the following formula (1) :

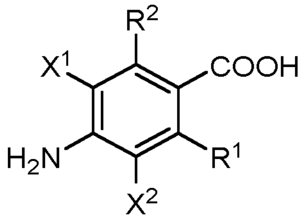

(1)

wherein $R^1$ represents a hydrogen atom, a hydroxy group, a methoxy group, an amino group, a fluorine atom, a chlorine atom, a bromine atom, an iodine atom, a carboxy group, a methyl group, or an ethyl group, $R^2$ represents a hydrogen atom, a hydroxy group, a methoxy group, an amino group, a fluorine atom, a chlorine atom, a bromine atom, an iodine atom, a carboxy group, a methyl group, or an ethyl group, and $X^1$ and $X^2$ each represent a hydrogen atom or a hydroxy group, at least one of which represents a hydroxy group.

<21> The method according to <20>, wherein the 4-amino-3-hydroxybenzoic acid derivatives are 4-amino-3-hydroxybenzoic acid derivatives, wherein both $R^1$ and $R^2$ are hydrogen atoms.

<22> The method according to any of <19> to <21>, wherein the 4-aminobenzoic acids are 4-aminobenzoic acid derivatives of the following formula (2):

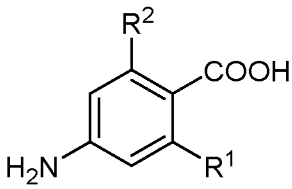

(2)

wherein $R^1$ represents a hydrogen atom, a hydroxy group, a methoxy group, an amino group, a fluorine atom, a chlorine atom, a bromine atom, an iodine atom, a carboxy group, a methyl group, or an ethyl group, and $R^2$ represents a hydrogen atom, a hydroxy group, a methoxy group, an amino group, a fluorine atom, a chlorine atom, a bromine atom, an iodine atom, a carboxy group, a methyl group, or an ethyl group.

<23> The method according to <22>, wherein the 4-aminobenzoic acid derivatives are 4-aminobenzoic acid derivatives, wherein both $R^1$ and $R^2$ are hydrogen atoms.

Examples

Hereinafter, the present invention will be described in more detail with reference to Examples. However, the present invention is not limited thereby.

Example 1 Production of 4-amino-3-hydroxybenzoic acid

In the following Example, PCR was performed using PrimeSTAR Max DNA Polymerase (Takara Bio Inc.) unless otherwise specified.

(1) Preparation of plasmid containing gene encoding wild-type enzyme

(a) Preparation of plasmid pECsf_gapS_pabABC

A DNA fragment containing genes encoding 4-amino-4-deoxychorismate synthase and 4-amino-4-deoxychorismate lyase was amplified by PCR using the genome extracted from a *Corynebacterium glutamicum* ATCC13032 strain by a routine method as a template and using primers GN14_127 (SEQ ID NO: 3, TATTAATTAAATGCGCGTTTTAATTATTGATAATTATGATTC) and GN14_133 (SEQ ID NO: 4, TTGCGGCCGCTTGTTTAAACCTCCTTACAGAAAAATGGTTGGGCG). This fragment was inserted between the PacI site and the NotI site of a plasmid pECsf_gapS (see Japanese Patent Application No. 2015-25491) to obtain a plasmid pECsf_gapS_pabABC.

(b) Preparation of plasmid pECsf_gapS_pabABC_HFM122

A DNA fragment for a vector was synthesized by PCR using the plasmid pECsf_gapS_pabABC obtained above as a template and using primers pabABCcory vec R (SEQ ID NO: 5, AAATTTAAACCTCCTTTACAGAAAAATGGTTGG) and pabABCcory vec F (SEQ ID NO: 6, GGAGGTTTAAACAAGCGGCCGCGATATC). Subsequently, a plasmid containing a gene (SEQ ID NO: 1) encoding a polypeptide HFM122 having 4-aminobenzoic acid hydroxylation activity was prepared by artificial gene synthesis, and a DNA fragment for an insert was synthesized by PCR using this plasmid as a template and using primers pECsfD HFM122 F (SEQ ID NO: 7, AGGAGGTTTAAATTTATGCGCACTCAGGTGGCTAT) and pECsfD HFM122 R (SEQ ID NO: 8, CTTGTTTAAACCTCCTTATACGAGTGGCAGTCCTA). These PCR products were treated with DpnI (Takara Bio Inc.). Then, the respective DNA fragments were purified using NucleoSpin Gel and PCR Clean-up (Takara Bio Inc.) and ligated using In-Fusion HD Cloning Kit (Takara Bio Inc.) to construct a plasmid pECsf_gapS_pabABC_HFM122. The ECOS Competent *E. coli* DH5α strain (Nippon Gene Co., Ltd.) was transformed with the obtained plasmid solution. The cell solution was spread over LBKm agar medium (1% Bacto Trypton, 0.5% yeast extract, 1% NaCl, 50 μg/mL kanamycin sulfate, 1.5% agar) and then left standing overnight at 37°C. The obtained colonies were subjected to PCR reaction using Sapphire Amp (Takara Bio Inc.) and primers pabABC+pobA for CPCR F (SEQ ID NO: 9, GCTATCAAAACATTCGGCACATTGGTTTTCC) and pabABC+pobA for CPCR R (SEQ ID NO: 10, GGAAGATGCGTGATCTGATCCTTCAACTC) to select a transformant confirmed to have the introduced DNA fragment of interest. The obtained transformant was inoculated to 2 mL of LBKm liquid medium (1% Bacto Trypton, 0.5% yeast extract, 1% NaCl, 50 μg/mL kanamycin sulfate) and cultured overnight at 37°C. A plasmid was purified from this culture solution using NucleoSpin Plasmid EasyPure (Takara Bio Inc.).

(c) Preparation of plasmid pECsf_gapS_pabABC_tuD_HFM122

A DNA fragment for a vector was synthesized by PCR using the plasmid pECsf_gapS_pabABC_HFM122 obtained above as a template and using primers pabC last R (SEQ ID NO: 11, TTACAGAAAAATGGTTGGGCGCAA) and HFM122 F (SEQ ID NO: 12, ATGCGCACTCAGGTGGCTATCG). Subsequently, a DNA fragment (SEQ ID NO: 13, TACGTACCTGCAGGTAGCGTGTCAGTAGGCGCGTAGGGTAAGTGGGGTAGCGGCTTG TTAGATATCTTGAAATCGGCTTTCAACAGCATTGATTTCGATGTATTTAGCTGGCCG TTACCCTGCGAATGTCCACAGGGTAGCTGGTAGTTTGAAAATCAACGCCGTTGCCCT TAGGATTCAGTAACTGGCACATTTTGTAATGCGCTAGATCTGTGTGCTCAGTCTTCC AGGCTGCTTATCACAGTGAAAGCAAAACCAATTCGTGGCTGCGAAAGTCGTAGCCAC CACGAAGTCCAAAGGAGGATCTAAATTATGAATAATATAAAAGGAGGAATTAATTAA ) containing tuf gene (cg0587) promoter (hereinafter, referred to as tu promoter) carried by a *Corynebacterium glutamicum* ATCC13032 strain was prepared by artificial gene synthesis, and a DNA fragment for an insert was synthesized by PCR using this fragment as a template and using primers pabC-Ptu F (SEQ ID NO: 14, ACCATTTTTCTGTAATACGTACCTGCAGGTAGCGTG) and Ptu-HFM122 R (SEQ ID NO: 15, CACCTGAGTGCGCATTTAATTAATTCCTCCTTTTA). These PCR products were treated with DpnI (Takara Bio Inc.). Then, the respective DNA fragments were purified using NucleoSpin Gel and PCR Clean-up (Takara Bio Inc.) and ligated using In-Fusion HD Cloning Kit (Takara Bio Inc.) to construct a plasmid pECsf_gapS_pabABC_tuD_HFM122. The ECOS Competent *E. coli* DH5α strain (Nippon Gene Co., Ltd.) was transformed with the obtained plasmid solution. The cell solution was spread over LBKm agar medium and then left standing overnight at 37°C. The obtained colonies were subjected to PCR reaction using Sapphire Amp (Takara Bio Inc.) and primers Ptu seq 1 (SEQ ID NO: 16, GCTTGTTAGATATCTTGAAATCGGCTTTC) and pabABC+pobA for CPCR R (SEQ ID NO: 10, GGAAGATGCGTGATCTGATCCTTCAACTC) to select a transformant confirmed to have the introduced DNA fragment of interest. The obtained transformant was inoculated to 2 mL of LBKm liquid medium and cultured overnight at 37°C. A plasmid was purified from this culture solution using NucleoSpin Plasmid EasyPure (Takara Bio Inc.).

In the constructed plasmid, the genes encoding 4-amino-4-deoxychorismate synthase and 4-amino-4-deoxychorismate lyase were linked under the control of gap promoter, and the gene encoding wild-type HFM122 was further linked under the control of the tu promoter.

(2) Preparation of plasmid containing gene encoding mutant enzyme

The preparation of a plasmid containing a gene encoding a mutant enzyme will be given below by taking, as an example, the preparation of a plasmid containing a gene encoding a mutant enzyme in which valine at position 39 of HFM122 was substituted with cysteine.

A plasmid pECsf_gapS_pabABC_tuD_HFM122_V39C was constructed by PCR using a plasmid pECsf_gapS_pabABC_tuD_HFM122 as a template and using complementary primers HFM122 V39C F (SEQ ID NO: 17, GCTTATTGTGAAGGCCGAGTTCGGGCT) and HFM122 V39C R (SEQ ID NO: 18, GCCTTCACAATAAGCACGGTCTTTGCG). The PCR product was treated with DpnI (Takara Bio Inc.). The ECOS Competent E. coli DH5α strain (Nippon Gene Co., Ltd.) was transformed with the solution thus treated. The cell solution was spread over LBKm agar medium and then left standing overnight at 37°C. The obtained colonies were selected as a transformant. The transformant was inoculated to 2 mL of LBKm liquid medium and cultured overnight at 37°C. A plasmid was purified from this culture solution using NucleoSpin Plasmid EasyPure (Takara Bio Inc.).

In a similar manner, a plasmid containing a gene encoding each enzyme mutant was obtained by PCR using

primers shown in the column "Primer" of Table 1 instead of the primers HFM122 V39C F and HFM122 V39C R.

[Table 1]

| Plasmid of interest | Primer | Sequence (5'→3 T) | SEQ ID NO |
|---|---|---|---|
| pECsf_gapS_pabABC_tu D_ HFM122_V43L | HFM122 V 43L F | GGCCGACTCCGGGCTG GTGTCCTGGAA | 19 |
| | HFM122 V 43L R | AGCCCGGAGTCGGCCT TCGACATAAGC | 20 |
| pECsf_gapS_pabABC_tu D_ HFM122_V43H | HFM122 V 43H F | GGCCGACATCGGGCTG GTGTCCTGGAA | 21 |
| | HFM122 V 43H R | AGCCCGATGTCGGCCT TCGACATAAGC | 22 |
| pECsf_gapS_pabABC_tu D_ HFM122_M83V | HFM122 M 83V F | GGCGAGGTTTTCCGTA TCGACATGGCA | 23 |
| | HFM122 M 83V R | ACGGAAAACCTCGCCA TCAGACGCAAG | 24 |
| pECsf_gapS_pabABC_tu D_ HFM122_M83G | HFM122 M 83G F | GGCGAGGGTTTCCGTA TCGACATGGCA | 25 |
| | HFM122 M 83G R | ACGGAAACCCTCGCCA TCAGACGCAAG | 26 |
| pECsf_gapS_pabABC_tu D_ HFM122_M83F | HFM122 M 83F F | GGCGAGTTCTTCCGTA TCGACATGGCA | 27 |
| | HFM122 M 83F R | ACGGAAGAACTCGCCA TCAGACGCAAG | 28 |
| pECsf_gapS_pabABC_tu D_ HFM122_M106A | HFM122 M 106A F | GAGGTGGCAAAGGACC TGTTTGATGCA | 29 |
| | HFM122 M 106A R | GTCCTTTGCCACCTCC TGTTGGCCGTA | 30 |
| pECsf_gapS_pabABC_tu D_ HFM122_M106C | HFM122 M 106C F | GAGGTGTGTAAGGACC TGTTTGATGCA | 31 |
| | HFM122 M 106C R | GTCCTTACACACCTCC TGTTGGCCGTA | 32 |
| pECsf_gapS_pabABC_tu D_ HFM122_M1061 | HFM122 M 106I F | GAGGTGATTAAGGACC TGTTTGATGCA | 33 |
| | HFM122 M 106I R | GTCCTTAATCACCTCC TGTTGGCCGTA | 34 |

(continued)

| Plasmid of interest | Primer | Sequence (5'→3 T) | SEQ ID NO |
|---|---|---|---|
| pECsf_gapS_pabABC_tu D_ HFM122_M106T | HFM122 M 106T F | GAGGTGACAAAGGACC TGTTTGATGCA | 35 |
| | HFM122 M 106T R | GTCCTTTGTCACCTCC TGTTGGCCGTA | 36 |
| pECsf_gapS_pabABC_tu D_ HFM122_V180A | HFM122 V 180A F | GAACGGGCATATCCCT TTGGGTGGTTG | 37 |
| | HFM122 V 180A R | GGGATATGCCCGTTCG AAGGTCTTGAG | 38 |
| pECsf_gapS_pabABC_tu D_ HFM122_A266V | HFM122 A 266V F | AGCATTGTTCCACTTC GCTCCTTCGTT | 39 |
| | HFM122 A 266V R | AAGTGGAACAATGCTC TTCTCGAAGGA | 40 |
| pECsf_gapS_pabABC_tu D_ HFM122_A266F | HFM122 A 266F F | AGCATTTTCCCACTTC GCTCCTTCGTT | 41 |
| | HFM122 A 266F R | AAGTGGGAAAATGCTC TTCTCGAAGGA | 42 |
| pECsf_gapS_pabABC_tu D_ HFM122_F346L | HFM122 F 346L F | TGGTGGCTCACCTCCC TTACTCACCGC | 43 |
| | HFM122 F 346L R | GGAGGTGAGCCACCAG CTGAAACGCTC | 44 |
| pECsf_gapS_pabABC_tu D_ HFM122_M363V | HFM122 M 363V F | CGCAAGGTTCAAGTCG CCGAATTGGCA | 45 |
| | HFM122 M 363V R | GACTTGAACCTTGCGG TCGAAGCCGTC | 46 |
| pECsf_gapS_pabABC_tu D_ HFM122_M363L | HFM122 M 363L F | CGCAAGCTCCAAGTCG CCGAATTGGCA | 47 |
| | HFM122 M 363L R | GACTTGGAGCTTGCGG TCGAAGCCGTC | 48 |
| pECsf_gapS_pabABC_tu D_ HFM122_I371F | HFM122 I 371F F | GCATACTTCAAGGGTT CTCGCGCTGCC | 49 |
| | HFM122 I 371F R | ACCCTTGAAGTATGCC AATTCGGCGAC | 50 |

(3) Introduction of plasmid into host cell

A *Corynebacterium glutamicum* DRHG145 strain (see Japanese Patent Application No. 2014-523757) was transformed with each plasmid obtained above by electroporation (Bio-Rad Laboratories, Inc.). The obtained transformed cell solution was spread over LBKm agar medium and then left standing at 30°C for 2 days. The obtained colonies were used as a transformant.

(4) Culture of transformant

Each transformant obtained above was inoculated to 600 μL of CGXII medium (containing 50 μg/mL kanamycin sulfate) shown in Table 2, and cultured at 30°C for approximately 48 hours. Then, bacterial cells were removed by centrifugation to obtain a culture supernatant. The concentration of 4-amino-3-hydroxybenzoic acid in the obtained culture supernatant was quantified in accordance with the method of Reference Example 1. The rate of improvement in the ability to produce 4-amino-3-hydroxybenzoic acid was calculated in accordance to the equation given below. In the equation, "WT" represents a "transformant into which the plasmid containing the gene encoding the wild-type enzyme is introduced", and "MT" represents a "transformant into which the plasmid containing the gene encoding the mutant enzyme is introduced".

(Equation 1)

Rate of improvement in production ability = Ability of MT to produce 4-amino-3-hydroxybenzoic acid / Ability of WT to produce 4-amino-3-hydroxybenzoic acid

[Table 2]

| CGXII medium composition (per L) | |
|---|---|
| Glucose | 50g |
| $(NH_4)_2SO_4$ | 20g |
| Urea | 5g |
| $KH_2PO_4$ | 1g |
| $K_2HPO_4$ | 1g |
| $MgSO_4 \cdot 7H_2O$ | 0.25g |
| $CaCl_2 \cdot 2H_2O$ | 10mg |
| $FeSO_4 \cdot 7H_2O$ | 10mg |
| $MnSO_4 \cdot 5H_2O$ | 10mg |
| $ZnSO_4 \cdot 7H_2O$ | 1mg |
| $CuSO_4 \cdot 5H_2O$ | 0.2mg |
| $NiCl_2 \cdot 6H_2O$ | 0.02mg |
| Biotin (pH7) | 0.2mg |
| Tryptone | 50g |

(5) Results

As shown in Table 3, the bacterial strain into which each mutant enzyme was introduced had the more improved ability to produce 4-amino-3-hydroxybenzoic acid than the bacterial strain into which the wild-type enzyme was introduced.

[Table 3]

| Hydroxylase | Ability to produce 4-amino-3-hydroxybenzoic acid (g/L) | Rate of improvement in production ability |
|---|---|---|
| HFM122 wt | 1.45 | 1.00 |
| HFM122 V39C | 2.31 | 1.60 |
| HFM122 V43L | 1.72 | 1.19 |

(continued)

| Hydroxylase | Ability to produce 4-amino-3-hydroxybenzoic acid (g/L) | Rate of improvement in production ability |
|---|---|---|
| HFM122 V43H | 2.70 | 1.86 |
| HFM122 M83V | 2.02 | 1.40 |
| HFM122 M83G | 2.16 | 1.49 |
| HFM122 M83F | 2.59 | 1.79 |
| HFM122 M106A | 2.71 | 1.87 |
| HFM122 M106C | 1.59 | 1.10 |
| HFM122 M106I | 2.71 | 1.87 |
| HFM122 M106T | 2.73 | 1.88 |
| HFM122 V180A | 2.77 | 1.92 |
| HFM122 A266F | 2.40 | 1.65 |
| HFM122 A266V | 2.73 | 1.89 |
| HFM122 F346L | 2.84 | 1.97 |
| HFM122 M363V | 2.49 | 1.72 |
| HFM122 M363L | 2.69 | 1.86 |
| HFM122 I371F | 2.77 | 1.92 |

Reference Example 1 Quantification of 4-amino-3-hydroxybenzoic acid

4-Amino-3-hydroxybenzoic acid was quantified by HPLC. A reaction solution to be subjected to HPLC analysis was appropriately diluted with 0.1% phosphoric acid. Then, insoluble matter was removed using AcroPrep 96-well filter plates (0.2 μm GHP membrane, Nihon Pall Ltd.).

The HPLC apparatus used was Chromaster (Hitachi High-Tech Science Corp.). The analytical column used was L-column CDS (4.6 mm I.D. × 150 mm, Chemicals Evaluation and Research Institute, Japan). Eluent A was a 0.1% phosphoric acid solution of 0.1 M potassium dihydrogen phosphate, and eluent B was 70% methanol. Gradient elution was performed under conditions involving a flow rate of 1.0 mL/min and a column temperature of 40°C. A UV detector (detection wavelength: 280 nm) was used for the detection of 4-amino-3-hydroxybenzoic acid. A concentration calibration curve was prepared using a standard sample [4-amino-3-hydroxybenzoic acid (distributor code A1194, Tokyo Chemical Industry Co., Ltd.)]. 4-Amino-3-hydroxybenzoic acid was quantified on the basis of the concentration calibration curve.

## Claims

**1.** A polypeptide having 4-aminobenzoic acid hydroxylation activity, consisting of the amino acid sequence represented by SEQ ID NO: 2 or an amino acid sequence having at least 90% identity thereto, and having an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 being the following amino acid:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

2. A method for producing a mutant polypeptide having 4-aminobenzoic acid hydroxylation activity, comprising substituting an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 with the following amino acid, in a polypeptide consisting of the amino acid sequence represented by SEQ ID NO: 2 or an amino acid sequence having at least 90% identity thereto, and having 4-aminobenzoic acid hydroxylation activity:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

3. A method for improving 4-aminobenzoic acid hydroxylation activity, comprising substituting an amino acid residue at position 39, 43, 83, 106, 180, 266, 346, 363 or 371 of the amino acid sequence represented by SEQ ID NO: 2 or a position corresponding to the position 39, 43, 83, 106, 180, 266, 346, 363 or 371 with the following amino acid, in a polypeptide consisting of the amino acid sequence represented by SEQ ID NO: 2 or an amino acid sequence having at least 90% identity thereto, and having 4-aminobenzoic acid hydroxylation activity:

(a) the position 39 or the position corresponding thereto: cysteine,
(b) the position 43 or the position corresponding thereto: leucine or histidine,
(c) the position 83 or the position corresponding thereto: valine, glycine, or phenylalanine,
(d) the position 106 or the position corresponding thereto: alanine, cysteine, isoleucine, or threonine,
(e) the position 180 or the position corresponding thereto: alanine,
(f) the position 266 or the position corresponding thereto: valine or phenylalanine,
(g) the position 346 or the position corresponding thereto: leucine,
(h) the position 363 or the position corresponding thereto: valine or leucine, and
(i) the position 371 or the position corresponding thereto: phenylalanine.

4. A polynucleotide encoding the polypeptide according to claim 1.

5. A vector or a DNA fragment comprising the polynucleotide according to claim 4.

6. A transformed cell comprising the vector or the DNA fragment according to claim 5.

7. The transformed cell according to claim 6, wherein the transformed cell is derived from *E. coli* or a bacterium of the genus *Corynebacterium.*

8. The transformed cell according to claim 6 or 7, wherein the transformed cell is a microorganism capable of supplying 4-aminobenzoic acids.

9. A method for producing 4-amino-3-hydroxybenzoic acids, comprising a step of culturing the transformed cell according to any one of claims 6 to 8.

10. The method according to claim 9, further comprising a step of collecting 4-amino-3-hydroxybenzoic acids from a culture medium.

11. The method according to claim 9 or 10, wherein the culture is performed in the presence of 4-aminobenzoic acids.

12. The method according to any one of claims 9 to 11, wherein

the 4-amino-3-hydroxybenzoic acids are 4-amino-3-hydroxybenzoic acid derivatives of the following formula (1) :

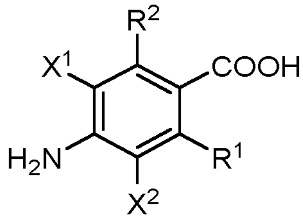

(1)

wherein $R^1$ represents a hydrogen atom, a hydroxy group, a methoxy group, an amino group, a fluorine atom, a chlorine atom, a bromine atom, an iodine atom, a carboxy group, a methyl group, or an ethyl group, $R^2$ represents a hydrogen atom, a hydroxy group, a methoxy group, an amino group, a fluorine atom, a chlorine atom, a bromine atom, an iodine atom, a carboxy group, a methyl group, or an ethyl group, and $X^1$ and $X^2$ each represent a hydrogen atom or a hydroxy group, at least one of which represents a hydroxy group.

**13.** The method according to claim 11 or 12, wherein

the 4-aminobenzoic acids are 4-aminobenzoic acid derivatives of the following formula (2):

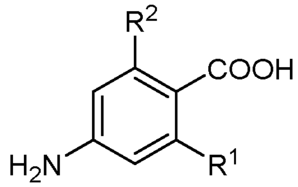

(2)

wherein $R^1$ represents a hydrogen atom, a hydroxy group, a methoxy group, an amino group, a fluorine atom, a chlorine atom, a bromine atom, an iodine atom, a carboxy group, a methyl group, or an ethyl group, and $R^2$ represents a hydrogen atom, a hydroxy group, a methoxy group, an amino group, a fluorine atom, a chlorine atom, a bromine atom, an iodine atom, a carboxy group, a methyl group, or an ethyl group.

## INTERNATIONAL SEARCH REPORT

International application No. **PCT/JP2021/031005**

**A. CLASSIFICATION OF SUBJECT MATTER**

***C12P 13/00***(2006.01)i; ***C12N 1/21***(2006.01)i; ***C12N 15/09***(2006.01)i; ***C12N 15/53***(2006.01)i; ***C12N 15/63***(2006.01)i; ***C12N 9/02***(2006.01)i
FI: C12N15/53 ZNA; C12N1/21; C12N9/02; C12N15/09 Z; C12N15/63 Z; C12P13/00

According to International Patent Classification (IPC) or to both national classification and IPC

**B. FIELDS SEARCHED**

Minimum documentation searched (classification system followed by classification symbols)

C12P13/00; C12N1/21; C12N15/09; C12N15/53; C12N15/63; C12N9/02

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

CAplus/MEDLINE/EMBASE/BIOSIS (STN); GenBank/EMBL/DDBJ/GeneSeq

**C. DOCUMENTS CONSIDERED TO BE RELEVANT**

| Category* | Citation of document, with indication, where appropriate, of the relevant passages | Relevant to claim No. |
|---|---|---|
| A | JP 2020-39330 A (KAO CORP.) 19 March 2020 (2020-03-19)<br>claims, examples | 1-13 |
| P, A | WO 2021/090925 A1 (KAO CORP.) 14 May 2021 (2021-05-14)<br>claims, examples | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 September 2021** | **28 September 2021** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (January 2015)

**INTERNATIONAL SEARCH REPORT**

International application No.
**PCT/JP2021/031005**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed:
      ☑ in the form of an Annex C/ST.25 text file.
      ☐ on paper or in the form of an image file.
   b. ☐ furnished together with the international application under PCT Rule 13*ter*.1(a) for the purposes of international search only in the form of an Annex C/ST.25 text file.
   c. ☐ furnished subsequent to the international filing date for the purposes of international search only:
      ☐ in the form of an Annex C/ST.25 text file (Rule 13*ter*.1(a)).
      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (January 2015)

**INTERNATIONAL SEARCH REPORT**
**Information on patent family members**

International application No.
**PCT/JP2021/031005**

| Patent document cited in search report | Publication date (day/month/year) | Patent family member(s) | Publication date (day/month/year) |
|---|---|---|---|
| JP 2020-39330 A | 19 March 2020 | WO 2020/054598 A1 | |
| WO 2021/090925 A1 | 14 May 2021 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 5445453 B
- JP 3821350 B
- JP 2018171849 A
- JP 2015025491 A
- JP 2014523757 A


### Non-patent literature cited in the description


- **HIROKI MURASE ; SENI GAKKAISHI ; SENI TO KOGYO.** *Journal of Fiber Science and Technology,* 2010, vol. 66 (6
- **LON J. MATHIAS et al.** *Macromolecules,* 1985, vol. 18 (4), 616-622
- **BARRIE ENTSCH et al.** *The Journal of Biological Chemistry,* 1987, vol. 262 (13), 6060-6068
- **DOMENICO L. GATTI et al.** *Biochemistry,* 1996, vol. 35 (2), 567-578
- *Science,* 1985, vol. 227, 1435-1441
- **THOMPSON, J.D. et al.** *Nucleic Acids Res.,* 1994, vol. 22, 4673-4680
- *Gene,* 1989, vol. 77 (1), 61-68
- *Nucleic Acids R4esearch,* 1989, vol. 17, 7059-7071
- **MCKENZIE, T. et al.** *Plasmid,* 1986, vol. 15 (2), 93-103
- **D.J. BALLANCE et al.** *Gene,* 1985, vol. 36, 321-331
- **VAN HARTINGSVELDT W et al.** *Mol Gen Genet,* 1987, vol. 206, 71-75
- **M.I.G. RONCERO et al.** *Gene,* 1989, vol. 84, 335-343
- **C.D. SKORY et al.** *Mol Genet Genomics,* 2002, vol. 268, 397-406
- **GRUBER, F. et al.** *Curr Genet,* 1990, vol. 18, 447-451